# EUROPEAN PATENT APPLICATION

(11) **EP 4 113 523 A1**
(43) Date of publication of application: **04.01.2023**
(21) Application number: 21182328.1
(22) Date of filing: 29.06.2021
(51) Int. Cl.: G16H 10/40, G16H 40/40

(54) **CONTROL RANGES FOR DEVICES FOR BIOLOGICAL SAMPLE ANALYSIS**

(71) Applicant: Radiometer Medical ApS, 2700 Brønshøj (DK)
(72) Inventor: DAMANI, Ruchi Bhupendra, 2700 Brønshøj (DK)
(74) Representative: Radiometer IPR

(57) **Abstract**

A computer-implemented method performed by a central system for establishing control ranges for a plurality of remote medical analyzer devices is disclosed. The method comprises obtaining a plurality of measurement results associated with a plurality of quality control (QC) measurements performed by a plurality of medical analyzer devices on a set of predefined QC samples. Each QC sample is associated with a predefined target range and wherein each medical analyzer device is associated with a peer group of medical analyzer devices. The method further comprises determining, for each peer group out of a plurality of peer groups, a peer control range for each predefined QC sample by processing the plurality of measurement results of the peer group in view of the associated target range. Furthermore, the method comprises determining, for each medical analyzer device, a control range for each predefined QC sample based on the determined peer control range of the associated peer group. The method further comprises transmitting, to a device for managing at least one medical analyzer device, data indicative of the determined control ranges for the set of predefined QC samples for the at least one medical analyzer device.

## Description

### TECHNICAL FIELD

The present disclosure relates generally to the field of clinical analysis and to devices configured to analyze biological samples. In particular, the present disclosure relates to quality control (QC) processes for such devices.

### BACKGROUND

Within the field of clinical analysis, a wide variety of electronic medical analyzers are known that allow clinical personnel to acquire test results and/or measurement results or otherwise analyze specimens such as samples of bodily fluids. These analyses include in vitro measurements on individual samples of e.g. whole blood, serum, plasma and urine, tissue samples or other types of samples obtained from a patient. Further, the analysis includes in vivo measurements on sample streams such as transcutaneous measurements of e.g. the partial pressures of oxygen (*p*O₂) and/or carbon dioxide (*p*CO₂) and also pulse oximetry measurements. Generally, a medical analyzer is a device configured to conduct chemical, optical, physical or similar analysis on specimen e.g. on individual samples or sample streams. Such medical analyzers include analyzers for performing various forms of clinical tests and/or analysis, such as the measurement of physiological parameters of a patient. In modern clinical environments, medical analyzers are widely used and there is a trend of moving more and more tests from a central laboratory to the actual point of care (POC).

Further, modern medical analyzers require regular quality control (QC), which may be understood as a process used to monitor and evaluate the analytical process that produces the patient results. There are a number of techniques to test the functionality of medical analyzers for QC purposes. However, a common practice is to perform periodic QC programs/processes where measurements are performed on a set of known standard samples (may also be referred to as control substances, QC specimen or QC samples), each having a predetermined characteristic, in order to verify that the measurement result falls within a predefined range of acceptable values for each QC sample. These predefined ranges are often referred to as a control ranges.

Currently, these control ranges are either obtained from the manufacturer (often referred to as manufacturer ranges) orfrom periodic internal testing. A problem with the manufacturer ranges is that they are often relatively wide, and therefore the QC programs/processes for testing the accuracy of the device, which are based on the manufacturer ranges, may have questionable reliability. Therefore, many facilities employ internal testing as part of their QC program for setting the control ranges in order to obtain more narrow ranges than the above mentioned manufacturer ranges.

However, a problem with the control ranges that are set by internal testing as part of each laboratory's QC program is that these processes are time consuming and procedurally inefficient. In more detail, such internal testing processes for establishing control ranges not only require valuable time from laboratory or healthcare professionals, but also result in that the analyzer is made unavailable for use during that time.

Thus, there is a need in the art for new solutions for establishing the control ranges for medical analyzers, which solve, or at least mitigate at least some of the above-discussed problems.

### SUMMARY

It should be emphasized that the term "comprises/comprising" (replaceable by "includes/including") when used in this specification is taken to specify the presence of stated features, integers, steps, or components, but does not preclude the presence or addition of one or more other features, integers, steps, components, or groups thereof. As used herein, the singular forms "a", "an" and "the" are intended to include the plural forms as well, unless the context clearly indicates otherwise.

Generally, when an apparatus or device is referred to herein, it is to be understood as a physical product. The physical product may comprise one or more parts, such as control circuitry in the form of one or more controllers, one or more processors, or the like.

It is an object of some embodiments to solve or mitigate, alleviate, or eliminate at least some of the above or other disadvantages.

A first aspect is a computer-implemented method performed by a central system for establishing control ranges for a plurality of remote medical analyzer devices. The method comprises obtaining a plurality of measurement results associated with a plurality of quality control (QC) measurements performed by a plurality of medical analyzer devices on a set of predefined QC samples. Each QC sample is associated with a predefined target range and wherein each medical analyzer device is associated with a peer group of medical analyzer devices. The method further comprises determining, for each peer group out of a plurality of peer groups, a peer control range for each predefined QC sample by processing the plurality of measurement results of the peer group in view of the associated target range. Furthermore, the method comprises determining, for each medical analyzer device, a control range for each predefined QC sample based on the determined peer control range of the associated peer group. The method further comprises transmitting, to a device for managing at least one medical analyzer device, data indicative of the determined control ranges for the set of predefined QC samples for the at least one medical analyzer device.

In some embodiments, the processing of the plurality of measurement results in view of the associated target ranges comprises discarding any measurement results having a difference value above a threshold in order to form a filtered set of measurement results. The difference value defines a difference between the measurement result and the corresponding predefined target range.

In some embodiments the processing of the plurality of measurement results in view of the associated target values further comprises determining, for each peer group, the peer control range based on a statistical function of the filtered set of measurement results.

In some embodiments the step of determining, for each peer group, the peer control range based on a statistical function of the filtered set of measurement results comprises determining, for each peer group, an average measurement value for each QC sample of the set of predefined QC samples, and determining, for each peer group, the peer control range for each QC sample based on the determined average measurement value and at least one standard deviation value.

In some embodiments, the step of determining, for each peer group, the average measurement value comprises determining, for each peer group, a moving average value for each QC sample over a most recent time period having a defined length.

In some embodiments, the method further comprises determining, for each medical analyzer device, an associated peer group out of a plurality of peer groups based on an analyzer type and QC product types of each medical analyzer device so that the medical analyzer devices that have a corresponding analyzer type and corresponding QC product types belong to the same peer group.

In some embodiments the step of determining, for each medical analyzer device, the control range for each predefined QC sample comprises computing each control range based on each corresponding peer control range, a number of measurement samples used to determine the corresponding peer control range, and a number of medical analyzer devices being comprised in the associated peer group.

Further, a second aspect is a (non-transitory) computer-readable storage medium storing one or more programs configured to be executed by one or more processors of a processing system, the one or more programs comprising instructions for performing the method according to any one of the embodiments disclosed herein. With this aspect, similar advantages and preferred features are present as in the previously discussed first aspect.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

A third aspect is an apparatus for establishing control ranges for a plurality of remote medical analyzer devices, the device comprises control circuitry configured to obtain a plurality of measurement results associated with a plurality of measurements performed by a plurality of medical analyzer devices on a set of predefined QC samples. Each QC sample is associated with a predefined target range and each medical analyzer device is associated with a peer group of medical analyzer devices. The control circuitry is further configured to determine, for each peer group, a peer control range for each predefined QC sample by processing the plurality of measurement results of the peer group in view of the associated target ranges. Furthermore, the control circuitry is configured to determine, for each medical analyzer device, a control range for each predefined QC sample based on the determined peer control ranges of the associated peer group. The control circuitry is further configured to transmit, to a device for managing at least one medical analyzer device, data indicative of the determined control ranges for the set of predefined QC samples for the at least one medical analyzer device. With this aspect, similar advantages and preferred features are present as in the previously discussed aspects and vice versa.

In some embodiments, the device for managing the medical analyzer device is configured to monitor and control one or more medical analyzer devices connected to the device.

In some embodiments, the device for managing the medical analyzer device is associated with a geographical location comprising one or more medical analyzer devices.

In some embodiments, the control circuitry is further configured to discard any measurement results having a difference value above a threshold in order to form a filtered set of measurement results. The difference value defines a difference between the measurement result and the corresponding predefined target range.

In some embodiments, the control circuitry is further configured to determine, for each peer group, the peer control range based on a statistical function of the filtered set of measurement results.

In some embodiments, the control circuitry is further configured to determine, for each peer group, an average measurement value for each QC sample of the set of predefined QC samples, and to determine, for each peer group, the peer control range for each QC sample based on the determined average measurement value and at least one standard deviation value.

In some embodiments, the control circuitry is further configured to determine, for each peer group, a moving average value for each QC sample over a most recent time period having a defined length.

In some embodiments, the control circuitry is further configured to determine, for each medical analyzer device, an associated peer group out of a plurality of peer groups based on an analyzer type and QC product types of each medical analyzer device so that the medical analyzer devices that have a corresponding analyzer type and corresponding QC product types belong to the same peer group.

In some embodiments, the control circuitry is further configured to compute each control range based on each corresponding peer control range, a number of measurement samples used to determine the corresponding peer control range, and a number of medical analyzer devices being comprised in the associated peer group.

A fourth aspect is a remote server comprising the apparatus according to any one of the embodiments of the third aspect disclosed herein. With this aspect, similar advantages and preferred features are present as in the previously discussed aspects and vice versa.

A fifth aspect is a cloud environment comprising one or more remote servers according to any one of the embodiments of the fourth aspect disclosed herein. With this aspect, similar advantages and preferred features are present as in the previously discussed aspects and vice versa.

A sixth aspect is a device for managing a plurality of medical analyzer devices. The device comprises control circuitry configured to obtain a plurality of measurement results associated with a plurality of QC measurements performed by the plurality of medical analyzer devices on a set of predefined QC samples. Each QC sample is associated with a predefined target range and each medical analyzer device is associated with a peer group of medical analyzer devices. The control circuitry is further configured to transmit, to a central agent (e.g. an apparatus according to the sixth aspect), data indicative of the obtained plurality of measurement results. Furthermore, the control circuitry is configured to receive, from the central agent, data indicative of a set of control ranges for the set of predefined QC samples for one or more peer groups associated with the plurality of medical analyzer devices. The control circuitry is further configured to configure, for each medical analyzer device, control ranges for the set of predefined QC samples, based on the received set of control ranges.

A seventh aspect is a system for establishing control ranges for a plurality of medical analyzer devices. The system comprises an apparatus according to any one of the embodiments of the third aspect and a device according to any one of the embodiments of the sixth aspect.

An advantage of some embodiments is that medical analyzer devices can be provided with narrow and precise control ranges in a more time effective and resource efficient manner.

An advantage of some embodiments is that detection of deviating medical analyzer devices is facilitated.

An advantage of some embodiments is that initialization/installation of new medical analyzer devices is facilitated as effective control ranges can be made available immediately.

Yet an advantage of some embodiments is that monitoring of control ranges in a group of medical analyzer devices is facilitated.

Yet an advantage of some embodiments is that monitoring of control ranges in a group of medical analyzer devices facilitates detection of even slightly deviating medical analyzer devices.

Yet an advantage of some embodiments is that monitoring of control ranges in a group of medical analyzer devices facilitates provision of narrow and precise control ranges.

These and other features and advantages of the embodiments described herein will in the following be further clarified with reference to the embodiments described hereinafter.

### BRIEF DESCRIPTION OF THE DRAWINGS

Further objects, features and advantages of some embodiments will appear from the following detailed description, reference being made to the accompanying drawings, in which:
Fig. 1 is schematic flowchart representation of a computer-implemented method performed by a central system for establishing control ranges for a plurality of remote medical analyzer devices in accordance with some embodiments.
Fig. 2 is a schematic block diagram representation of a system having an apparatus for establishing control ranges for a plurality of remote medical analyzer devices in accordance with some embodiments.
Fig. 3 is a schematic block diagram representation of a system having an apparatus for establishing control ranges for a plurality of remote medical analyzer devices in accordance with some embodiments.

### DETAILED DESCRIPTION

Those skilled in the art will appreciate that the steps, services and functions explained herein may be implemented using individual hardware circuitry, using software functioning in conjunction with a programmed microprocessor or general purpose computer, using one or more Application Specific Integrated Circuits (ASICs) and/or using one or more Digital Signal Processors (DSPs). It will also be appreciated that when embodiments are described in terms of a method, it may also be embodied in one or more processors and one or more memories coupled to the one or more processors, wherein the one or more memories store one or more programs that perform the steps, services and functions disclosed herein when executed by the one or more processors.

Embodiments of the present disclosure will be described and exemplified more fully hereinafter with reference to the accompanying drawings. The solutions disclosed herein can, however, be realized in many different forms and should not be construed as being limited to the embodiments set forth herein.

In the following, embodiments will be described for establishing narrow and effective control ranges for a plurality of distributed medical analyzer devices by utilizing peer group data to compute suitable control ranges.

Generally, when a medical analyzer device is referred to herein, it may be understood as a device configured to conduct chemical, optical, physical or similar analysis on specimen e.g. on individual samples or sample streams. Such medical analyzer devices include analyzers for performing various forms of clinical tests and/or analysis, such as the measurement of physiological parameters of a patient.

Generally, when a "device for managing one or more medical analyzer devices" is referred to herein, it may be understood as a device associated with the hospital or facility comprising the one or more medical analyzer devices. However, the one or more medical analyzer devices may be distributed over a number of geographical locations, wherefore the "device for managing one or more medical analyzer devices" may also be associated with a plurality of hospitals or facilities comprising the one more medical analyzer devices. The "device for managing one or more medical analyzer devices" is configured to keep an overview or monitor a status and operation of the one or more medical analyzer devices. Moreover, the "device for managing one or more medical analyzer devices" is configured to transmit data (e.g. instructions) to the medical analyzer devices associated therewith.

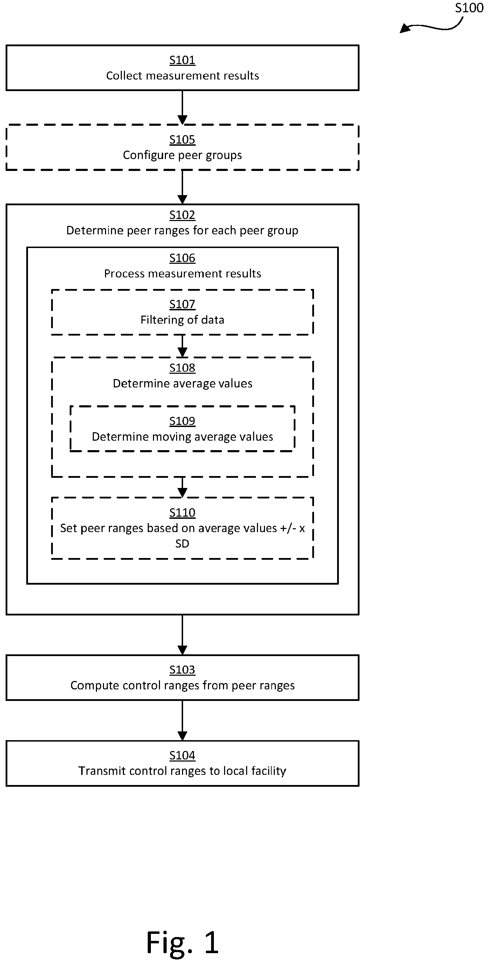

Fig. 1 is a schematic flowchart representation of a computer-implemented method S100 performed by a central system (central agent) for establishing control ranges for a plurality of remote medical analyzer devices. A control range may be understood as a range within which a Quality Control (QC) parameter should fall. The control ranges are specific for specific QC measurement parameters, where each measurement parameter is associated with a specific QC sample (may also be known as QC specimen or QC liquid).

Some examples of QC measurement parameters include, but are not limited to - pH (measure of the acidity of alkalinity of a sample) - *p*CO₂ (partial pressure (or tension) of carbon dioxide in blood) - *p*O₂ (partial pressure (or tension) of oxygen in blood) - ctHb (concentration of total hemogloblin in blood) - sO₂ (oxygen saturation) - *F*O₂Hb (fraction of oxyhemoglobin in total hemoglobin in blood) - FCOHb (fraction of carboxyhemoglobin in total hemoglobin in blood) - FMetHb (fraction of methemoglobin in total hemoglobin in blood) - cNa⁺ (concentration of sodium ions in plasma) - cGlu (concentration of D-glucose in plasma). This list should not be construed as exhaustive but may include further measurement parameters which are readily understood by the skilled person in the art.

Moving on, the method comprises obtaining S101 a plurality of measurement results associated with a plurality of QC measurements performed by a plurality of medical analyzer devices on a set of a predefined QC samples. Each QC sample is associated with a predefined target range and wherein each medical analyzer device is associated with a peer group of medical analyzer devices. In more detail, a QC sample may understood as a substance or sample having a measurable property that is carefully controlled such that when a medical analyzer devices performs a measurement on the QC sample to verify its operational accuracy, there is an associated target range (e.g. target value ± tolerance value) that the measurement result within which the result should fall in order to fulfil the associated quality requirements. The target ranges may for example be set by the manufacturer or distributor of the QC samples. The term obtaining is herein to be interpreted broadly and encompasses receiving, retrieving, collecting, acquiring, determining, deriving, and so forth.

The method further comprises determining, for each peer group out of a plurality of peer groups, a peer control range for each predefined QC sample by processing S106 the obtained S101 plurality of measurement results of the peer group in view of the associated target range.

In general, a peer group of medical analyzer devices as referred to herein, may be understood as a group of medical analyzer devices that share some specific common traits. For example, a peer group of medical analyzer devices may be defined as medical analyzer devices of the same type (e.g. same model) and/or as medical analyzer devices having the same QC product type (i.e. using QC samples originating from the same source). Furthermore, the peer groups may further comprise geographical constraints - e.g. even if the medical analyzer devices are of the same type they need not necessarily belong to the same peer group if they are associated with (e.g. residing in) different countries.

Thus, in some embodiments, the method S100 may further comprise obtaining data indicative of a medical analyzer type and/or a QC product type from each of the plurality of medical analyzer devices. The data may for example be transmitted as metadata together with the measurement results. Accordingly, in some embodiments, the method S100 further comprises determining S105, for each medical analyzer device, an associated peer group out of a plurality of peer groups based on an analyzer type and QC product types of each medical analyzer device so that the medical analyzer devices that have a corresponding analyzer type and corresponding QC product types belong to the same peer group.

In general, the "measurement results of the peer group" may be understood as previously obtained measurement results from the peer group, i.e. one or more previously obtained measurement results. In some embodiments, the "measurement results of the peer group" comprise an average measurement result for each QC measurement of the associated peer group. Moreover, in some embodiments, the "measurement results of the associated peer group" comprises a moving average measurement result for each QC measurement of the peer group.

Further, in reference to the processing S106 of the measurement results, in some embodiments, the processing S106 of the obtained measurement results comprises discarding/filtering S107 any measurement results having a difference value above a threshold in order to form a filtered set of measurement results, wherein the difference value defines a difference between the measurement result and the corresponding predefined target range. Thereby, statistical outliers in the measurement results may be removed, and the reliability of the determined control ranges may be increased.

Moreover, in some embodiments, the processing S106 of the plurality of measurement results further comprises, determining, for each peer group, the peer control range based on at least one statistical function of the filtered set of measurement results. The at least one statistical function may for example comprise "mean function" of the measurement results, i.e. a function where the output of the function is an average value (may also be referred to as the mean) of the measurement results. The statistical function may comprise a "median function", i.e. a function where the output of the function is a median value of the measurement results. The statistical function may comprise "standard deviation function", i.e. a function where the output of the function is a standard deviation of the measurement results.

Thus, in some embodiments, the step of determining, for each peer group, the peer control range based on a statistical function of the filtered set of measurement results comprises determining S108, for each peer group, an average measurement value for each QC sample of the set of predefined QC samples, and determining S110, for each peer group, the peer control range for each QC sample based on the determined average measurement value and at least one standard deviation value.

Furthermore, in some embodiments, the determining, for each peer group, the average measurement value comprises determining S109, for each peer group, a moving average value for each QC sample over a most recent time period having a defined length (e.g. 2 -12 months). In other words, the average measurement value may be a rolling average value, such as e.g. a 6-month rolling average, a 5-month rolling average, or any other suitable length.

Moving on, once the peer control range(s) has/have been determined S102, the method S100 further comprises, determining S103, for each medical analyzer device, a control range for each predefined QC sample based on the determined S102 peer control range. In more detail, the determination S103 of the control ranges may comprise computing S103 each control range based on each corresponding peer control range, a number of measurement samples used to determine the corresponding peer control range, and a number of medical analyzer devices being comprised in the associated peer group.

Further, the method S100 comprises transmitting S104, to a device for managing at least one medical analyzer device, data indicative of the determined S103 control range for the set of predefined QC samples for the at least one medical analyzer device. Thus, each medical analyzer device is provided with a centrally computed control ranges that are based on the peer control ranges of an associated peer group of medical analyzer devices.

Accordingly, the medical analyzer devices are provided with the desirable narrow control ranges without requiring the medical analyzer devices to run through a time consuming internal testing as part of the QC program. Moreover, the control ranges as provided herein are based on a peer control ranges (which are based on measurement results from a number of "peer" devices), which provide a redundancy and reliability to the computed control ranges.

Executable instructions for performing these functions are, optionally, included in a non-transitory computer-readable storage medium or other computer program product configured for execution by one or more processors.

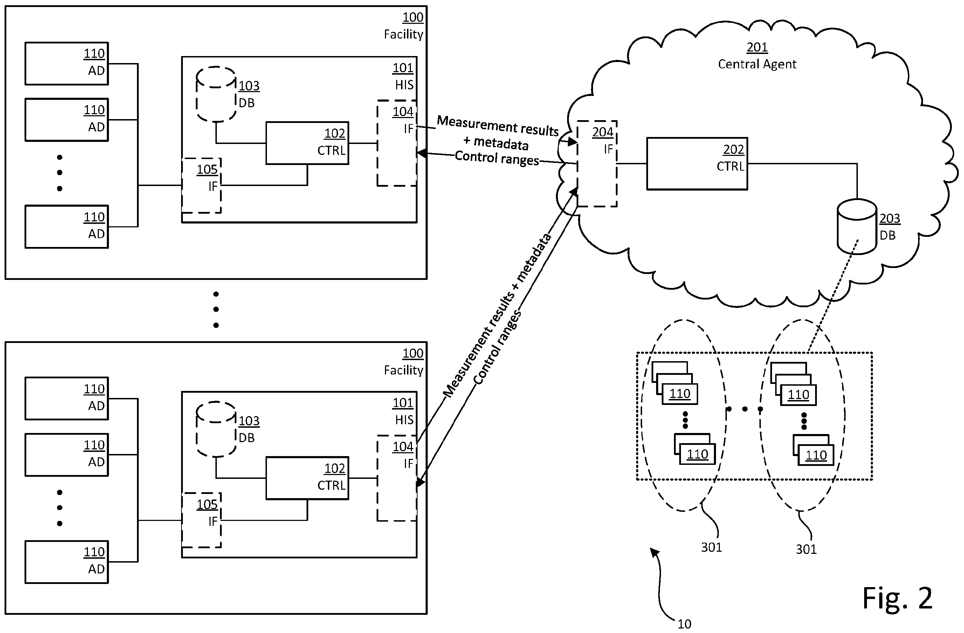

Fig. 2 is a schematic block diagram representation of a system 10 comprising an apparatus 201 for establishing control ranges for a plurality of remote medical analyzer devices 110 in accordance with some embodiments. The apparatus 201 comprises control circuitry CTRL 202 configured to obtain a plurality of measurement results associated with a plurality of quality control (QC) measurements performed by a plurality of medical analyzer devices 110 on a plurality of predefined QC samples. Each QC sample is associated with a predefined target range and each medical analyzer device 110 is associated with a peer group 301 of medical analyzer devices.

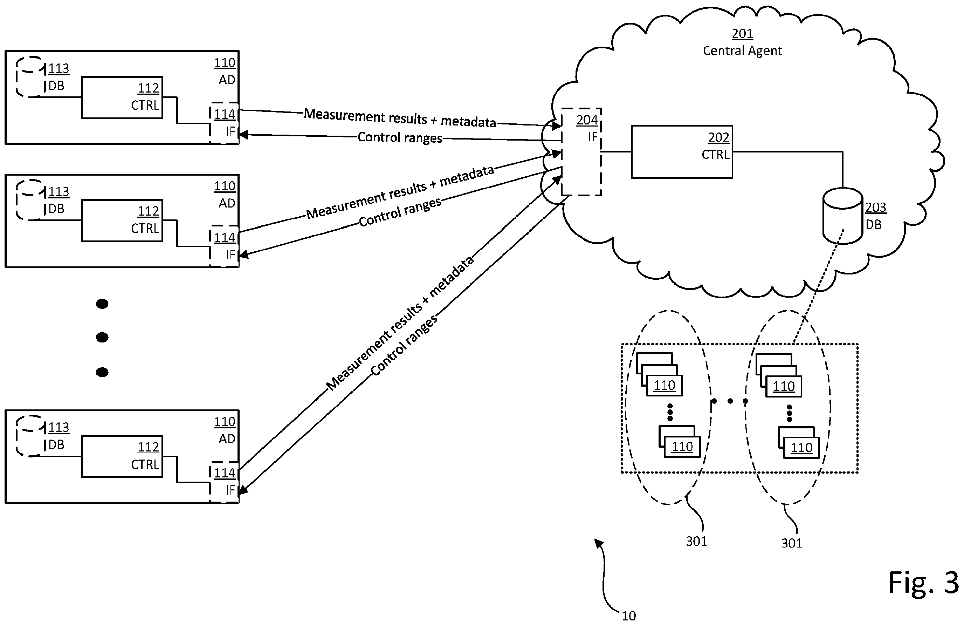

Further, the control circuitry CTRL 202 is configured to determine, for each peer group (e.g., a peer group of medical analyzer devices as indicated by the broken-line circles 301 in Figures 2 and 3), a peer control range for each predefined QC sample by processing the obtained plurality of measurement results of the peer group in view of the associated target ranges. The control circuitry 202 is further configured to determine, for each medical analyzer device of a plurality of medical analyzer devices, a control range for each predefined QC sample based on the determined peer control ranges of the associated peer group.

The control circuitry 202 may further be configured to obtain data (e.g. metadata associated with the measurement results) indicative of an analyzer type and QC product types of each medical analyzer device. This data (e.g. metadata) may subsequently be used to determine, for each medical analyzer device, an associated peer group out of a plurality of peer groups based on the analyzer type and the QC product types of each medical analyzer device so that the medical analyzer devices that have a corresponding analyzer type and corresponding QC product types belong to the same peer group.

Still further, the control circuitry 202 is configured to transmit, to a device 101, for managing at least one medical analyzer device 110, data indicative of the determined control ranges for the set of predefined QC samples for the at least one medical analyzer device. In other words, the control circuitry 202 determines a set of control ranges for each medical analyzer device 110 based on the peer control ranges of the associated peer group, and subsequently pushes the determined set of control ranges to the medical analyzer devices 110.

In some embodiments, the device 101 for managing the medical analyzer device has control circuitry 102 configured to monitor and control one or more medical analyzer devices connected to the device. Thus, the device 101 for managing the medical analyzer devices may be part of a Hospital Information System (HIS) and/or a Laboratory Information System (LIS), responsible for monitoring and controlling a plurality of medical analyzer devices associated thereto. Thus, in some embodiments, the device 101 for managing the medical analyzer device is associated with a geographical location comprising one or more medical analyzer devices. Nevertheless, in some embodiments, the device may be in the form of (e.g., implemented as) a cloud service (provided by a cloud environment, e.g., a cloud computing system, comprising one or more remote servers, e.g., distributed cloud computing resources) communicatively connected to one or more medical analyzer devices.

However, in some embodiments, the control circuitry 202 may be configured to transmit the data indicative of the determined control ranges for the set of predefined QC samples directly to each medical analyzer device 110. Thus, in some embodiments, the device 101 for managing the medical analyzer device may be an internal control unit or control circuitry CTRL 112 of the medical analyzer device 110, as for example depicted in Fig. 3, which is a schematic block diagram representation of a system comprising an apparatus 201 for establishing control ranges for a plurality of remote medical analyzer devices 110.

Reverting back to Fig. 2, the device 101 for managing a plurality of medical analyzer devices 110 has control circuitry 102 configured to obtain a plurality of measurement results associated with a plurality of QC measurements performed by the plurality of medical analyzer devices 110 on a set of predefined QC samples. The plurality of medical analyzer and the device 101 may for example be associated with the same facility (e.g. hospital or laboratory). Moreover, each QC sample is associated with a predefined target range and each medical analyzer device is associated with a peer group of medical analyzer devices.

Further, the control circuitry 102 is configured to transmit, to a central agent 201 (e.g. the apparatus described in the foregoing), data indicative of the obtained plurality of measurement results. As mentioned, the control circuitry 102 may be further configured to transmit data indicative of the analyzer type and the QC product types of each medical analyzer device of the plurality of medical analyzer devices.

The control circuitry 102 is further configured to receive, from the central agent 201, data indicative of a set of control ranges for the set of predefined QC samples for one or more peer groups associated with the plurality of medical analyzer devices 110. Further, the control circuitry is configured to configure, for each medical analyzer device of the plurality of medical analyzer devices, control ranges for the set of predefined QC samples based on the received set of control ranges.

Other embodiments than the above-described are possible and within the scope of the claims. Different method steps than those described above, performing the method by hardware or software, may be provided within the scope of the embodiments disclosed herein. Thus, according to an exemplary embodiment, there is provided a non-transitory computer-readable storage medium storing one or more programs configured to be executed by one or more processors of a processing system, the one or more programs comprising instructions for performing the method according to any one of the above-discussed embodiments. Alternatively, according to another exemplary embodiment a cloud computing system can be configured to perform any of the methods presented herein. The cloud computing system may comprise distributed cloud computing resources that jointly perform the methods presented herein under control of one or more computer program products.

Generally speaking, a computer-accessible medium may include any tangible or non-transitory storage media or memory media such as electronic, magnetic, or optical media-e.g., disk or CD/DVD-ROM coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 102, 112, 202 (associated with the devices and apparatus 101, 110, 201) may be or include any number of hardware components for conducting data or signal processing or for executing computer code stored in memory 103, 113, 203. The devices and apparatuses 101, 110, 201 have associated memory 103, 113, 203 and the memory 103, 113, 203 may be one or more devices for storing data and/or computer code for completing or facilitating the various methods described in the present description. The memory may include volatile memory or non-volatile memory. The memory 103, 113, 203 may include database components, object code components, script components, or any other type of information structure for supporting the various activities of the present description. According to an exemplary embodiment, any distributed or local memory device may be utilized with the apparatuses and methods of this description. According to an exemplary embodiment the memory 103, 113, 203 is communicably connected to the processor 102, 112, 202 (e.g., via a circuit or any other wired, wireless, or network connection) and includes computer code for executing one or more processes described herein.

The communication interfaces 104, 105, 114, 204 may provide the possibility to send output to a remote location (e.g. remote operator or control centre) by means of a suitable communication arrangement. The communication interfaces 104, 105, 114, 204 may be arranged to communicate with other functional components and may thus be seen as control interface also; however, a separate control interface (not shown) may be provided. Local communication within the facility 100 may also be of a wireless type with protocols such as WiFi, LoRa, Zigbee, Bluetooth, or similar mid/short range technologies.

Accordingly, it should be understood that parts of the described solution may be implemented either in a remote server, or in plurality of remote servers, for instance in a plurality of servers in communication with each other, a so called cloud solution. The different features and steps of the embodiments may be combined in other combinations than those described.

Generally, all terms used herein are to be interpreted according to their ordinary meaning in the relevant technical field, unless a different meaning is clearly given and/or is implied from the context in which it is used.

It should be noted that the word "comprising" does not exclude the presence of other elements or steps than those listed and the words "a" or "an" preceding an element do not exclude the presence of a plurality of such elements. It should further be noted that any reference signs do not limit the scope of the claims, that the embodiments may be at least in part implemented by means of both hardware and software, and that several "control circuits", "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the disclosed embodiments. Likewise, software implementations could be accomplished with standard programming techniques with rule-based logic and other logic to accomplish the various connection steps, processing steps, comparison steps and decision steps. The above mentioned and described embodiments are only given as examples and should not be limiting to the present disclosure. Hence, it should be understood that the details of the described embodiments are merely examples brought forward for illustrative purposes, and that all variations that fall within the scope of the claims are intended to be embraced therein.

## Claims

1. A computer-implemented method (S100) performed by a central system for establishing control ranges for a plurality of remote medical analyzer devices, the method (S100) comprising:
obtaining (S101) a plurality of measurement results associated with a plurality of quality control, QC, measurements performed by a plurality of medical analyzer devices on a set of predefined QC samples, wherein each QC sample is associated with a predefined target range and wherein each medical analyzer device is associated with a peer group of medical analyzer devices;
determining (S102), for each peer group out of a plurality of peer groups, a peer control range for each predefined QC sample by processing (S106) the plurality of measurement results of the peer group in view of the associated target range;
determining (S103), for each medical analyzer device, a control range for each predefined QC sample based on the determined (S102) peer control range of the associated peer group; and
transmitting (S104), to a device for managing at least one medical analyzer device, data indicative of the determined (S103) control ranges for the set of predefined QC samples for the at least one medical analyzer device.

2. The method (S100) according to claim 1, wherein the processing (S106) of the plurality of measurement results in view of the associated target ranges comprises:
discarding (S107) any measurement results having a difference value above a threshold in order to form a filtered set of measurement results, wherein the difference value defines a difference between the measurement result and the corresponding predefined target range.

3. The method (S100) according to claim 2, wherein the processing (S106) of the plurality of measurement results in view of the associated target values further comprises:
determining, for each peer group, the peer control range based on a statistical function of the filtered set of measurement results.

4. The method (S100) according to claim 3, wherein the step of determining, for each peer group, the peer control range based on a statistical function of the filtered set of measurement results comprises:
determining (S108), for each peer group, an average measurement value for each QC sample of the set of predefined QC samples;
determining (S110), for each peer group, the peer control range for each QC sample based on the determined average measurement value and at least one standard deviation value.

5. The method (S100) according to claim 4, wherein the determining, for each peer group, the average measurement value comprises:
determining (S109), for each peer group, a moving average value for each QC sample over a most recent time period having a defined length.

6. The method (S100) according to any one of claims 1-5, further comprising:
determining (S105), for each medical analyzer device, an associated peer group out of a plurality of peer groups based on an analyzer type and QC product types of each medical analyzer device so that the medical analyzer devices that have a corresponding analyzer type and corresponding QC product types belong to the same peer group.

7. The method according to any one of claims 1-6, wherein the step of determining (S103), for each medical analyzer device, the control range for each predefined QC sample comprises:
computing (S103) each control range based on each corresponding peer control range, a number of measurement samples used to determine the corresponding peer control range, and a number of medical analyzer devices being comprised in the associated peer group.

8. A computer-readable storage medium storing one or more programs configured to be executed by one or more processors of a processing system, the one or more programs comprising instructions for performing the method according to any one of claims 1-7.

9. An apparatus (201) for establishing control ranges for a plurality of remote medical analyzer devices (110), the device comprising control circuitry (202) configured to:
obtain a plurality of measurement results associated with a plurality of measurements performed by a plurality of medical analyzer devices (110) on a set of predefined QC samples, wherein each QC sample is associated with a predefined target range and wherein each medical analyzer device is associated with a peer group of medical analyzer devices;
determine, for each peer group, a peer control range for each predefined QC sample by processing the plurality of measurement results of the peer group in view of the associated target ranges;
determine, for each medical analyzer device, a control range for each predefined QC sample based on the determined peer control ranges of the associated per group; and
transmit, to a device for managing at least one medical analyzer device (110), data indicative of the determined control ranges for the set of predefined QC samples for the at least one medical analyzer device.

10. The apparatus according to claim 9, wherein the device for managing the medical analyzer device is configured to monitor and control one or more medical analyzer devices connected to the device.

11. The apparatus according to any of claims 9 - 10, wherein the device for managing the medical analyzer device is associated with a geographical location comprising one or more medical analyzer devices.

12. A remote server comprising the apparatus according to any one of claims 9 - 11.

13. A cloud environment comprising one or more remote servers according to claim 12.

14. A device (101) for managing a plurality of medical analyzer devices (110), the device comprising control circuitry (102) configured to:
obtain a plurality of measurement results associated with a plurality of QC measurements performed by the plurality of medical analyzer devices (110) on a set of predefined QC samples, wherein each QC sample is associated with a predefined target range and wherein each medical analyzer device is associated with a peer group of medical analyzer devices;
transmit, to a central agent (201), data indicative of the obtained plurality of measurement results;
receive, from the central agent (201), data indicative of a set of control ranges for the set of predefined QC samples for one or more peer groups associated with the plurality of medical analyzer devices (110);
configure, for each medical analyzer device (110), control ranges for the set of predefined QC samples, based on the received set of control ranges.

15. A system (10) for establishing control ranges for a plurality of medical analyzer devices (110), the system comprising:
an apparatus (201) according to any one of claims 9 - 13; and
a device (101) according to claim 14.
